# EUROPÄISCHE PATENTANMELDUNG

(11) **EP 1 090 998 A1**
(43) Veröffentlichungstag der Anmeldung: **11.04.2001**
(21) Anmeldenummer: 00121158.0
(22) Anmeldetag: 29.09.2000
(51) Int. Cl.: C12N 15/60, C12N 9/88, C12P 13/08

(54) **Für das eno-Gen codierende Nukleotidsequenzen**

(30) Priorität: 05.10.1999 DE 19947791
(71) Anmelder: Degussa-Hüls Aktiengesellschaft, 60287 Frankfurt am Main (DE)
(72) Erfinder: Möckel, Bettina, Dr., 40597 Düsseldorf (DE); Pfefferle, Walter, Dr., 33790 Halle (Westf.) (DE); Hermann, Thomas, Dr., 33739 Bielefeld (DE); Pühler, Alfred, Prof., 33739 Bielefeld (DE); Kalinowski, Jörn, Dr., 33615 Bielefeld (DE); Bathe, Brigitte, Dr., 33154 Salzkotten (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c),
und Verfahren zur fermentativen Herstellung von L-Aminosäuren unter Verstärkung des eno-Gens.

## Beschreibung

Gegenstand der Erfindung sind für das eno-Gen kodierende Nukleotidsequenzen und Verfahren zur fermentativen Herstellung von Aminosäuren, insbesondere L-Lysin unter Verwendung von coryneformen Bakterien, in denen das eno-Gen verstärkt wird.

### Stand der Technik

Aminosäuren, insbesondere L-Lysin finden in der Humanmedizin und in der pharmazeutischen Industrie insbesondere aber in der Tierernährung Anwendung.

Es ist bekannt, daß Aminosäuren durch Fermentation von Stämmen coryneformer Bakterien insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Aminosäuren sind und L-Lysin produzieren.

Seit einigen Jahren werden ebenfalls Methoden der rekombinanten DNA-Technik zur Stammverbesserung Aminosäure produzierender Stämme von Corynebacterium eingesetzt, indem man einzelne Aminosäure-Biosynthesegene amplifiziert und die Auswirkung auf die Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von Aminosäuren insbesondere L-Lysin bereitzustellen.

### Beschreibung der Erfindung

Aminosäuren insbesondere L-Lysin finden in der Humanmedizin, in der pharmazeutischen Industrie und insbesondere in der Tierernährung Anwendung. Es besteht daher ein allgemeines Interesse daran, neue verbesserte Verfahren zur Herstellung von Aminosäuren insbesondere L-Lysin bereitzustellen.

Wenn im folgenden L-Lysin oder Lysin erwähnt werden, sind damit nicht nur die Base sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ-ID-No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

Weitere Gegenstände sind
ein Polynukleotid gemäß Anspruch 4, enthaltend die Nukleotidsequenz wie in SEQ ID No. 1 dargestellt,
ein Polynukleotid gemäß Anspruch 6, das für ein Polypeptid codiert, das die Aminosäuresequenz, wie in SEQ ID No. 2 dargestellt, enthält
ein Vektor, enthaltend das Polypeptid gemäß Anspruch 1, insbesondere Pendelvektor oder Plasmidvektor
und als Wirtszelle dienende coryneforme Bakterien, die den Vektor enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthält, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind geeignet als Hybridisierungs-Sonden für RNA, cDNA und DNA, um cDNA in voller Länge zu isolieren, die für Enolase codieren und solche cDNA oder Gene zu isolieren, die eine hohe Ähnlichkeit der Sequenz mit der des Enolase-Gens aufweisen.

Polynukleotidsequenzen gemäss der Erfindung sind weiterhin geeignet als Primer zur Herstellung von DNA von Genen, die für Enolase codieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Basen. Geeignet sind ebenfalls Oligonukleotide mit einer Länge von mindestens 40 oder 50 Basenpaaren.

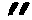 Isoliert" bedeutet aus seinem natürlichen Umfeld herausgetrennt.

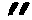 Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter 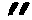 Polypeptiden" versteht man Peptide oder Proteine, die zwei oder mehr über Peptidbindungen verbundene Aminosäuren enthalten.

Die Polypeptide gemäß Erfindung schliessen ein Polypeptid gemäß SEQ ID No. 2, insbesondere solche mit der biologischen Aktivität der Enolase und auch solche ein, die zu wenigstens 70 % identisch sind mit dem Polypeptid gemäss SEQ ID No. 2, bevorzugt zu wenigstens 80% und besonders die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von Aminosäuren insbesondere L-Lysin unter Verwendung von coryneformen Bakterien, die insbesondere bereits eine Aminosäure produzieren, und in denen die für das eno-Gen codierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren, insbesondere L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die zum Beispiel bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715.

Den Erfindern gelang es, das neue, für das Enzym Enolase (EC 4.2.1.11) kodierende eno-Gen von C. glutamicum zu isolieren.

Zur Isolierung des eno-Gens oder auch anderer Gene von C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Viera et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Auf diese Weise wurde die neue für das Gen eno kodierende DNA-Sequenz von C. glutamicum erhalten, die als SEQ ID NO 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID NO 2 ist die sich ergebende Aminosäuresequenz des eno-Genproduktes dargestellt.

Kodierende DNA-Sequenzen, die sich aus SEQ ID NO 1 durch die Degeneriertheit des genetischen Codes ergeben, sind ebenfalls Bestandteil der Erfindung. In gleicher Weise sind DNA-Sequenzen, die mit SEQ ID NO 1 oder Teilen von SEQ ID NO 1 hybridisieren Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als 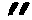 Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 ergeben sind ebenfalls Bestandteil der Erfindung.

In gleicher Weise sind DNA-Sequenzen, die mit SEQ ID NO 1 oder Teilen von SEQ ID NO 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID NO 1 ergeben. Derartige Oligonukleotide haben typischerweise eine Länge von mindestens 15 Basenpaaren.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des eno-Gens in verbesserter Weise Aminosäuren insbesondere L-Lysin produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße eno-Gen mit Hilfe von Plasmiden überexprimiert. Als Plasmide eignen sich solche die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Zusätzlich kann es für die Produktion von Aminosäuren, insbesondere L-Lysin vorteilhaft sein, neben dem eno-Gen eines oder mehrere Enzyme des jeweiligen Biosyntheseweges, der Glykolyse, der Anaplerotik, des Zitronensäure-Zyklus oder des Aminosäure-Exports zu überexprimieren.

So kann beispielsweise für die Herstellung von L-Lysin
- gleichzeitig das für die Dihydrodipicolinat-Synthase kodierende dapA-Gen (EP-B 0 197 335), oder
- gleichzeitig das für die Glyceraldehyd-3-Phosphat Dehydrogenase kodierende gap-Gen (Eikmanns (1992). Journal of Bacteriology 174:6076-6086), oder
- gleichzeitig das für die Triosephosphat Isomerase codierende tpi-Gen(Eikmanns (1992). Journal of Bacteriology 174:6076-6086), oder
- gleichzeitig das für die 3-Phosphoglycerat Kinase codierende pgk-Gen(Eikmanns (1992). Journal of Bacteriology 174:6076-6086), oder
- gleichzeitig das für die Pyruvat Carboxylase codierende pyc-Gen(Eikmanns (1992). Journal of Bacteriology 174:6076-6086), oder
- gleichzeitig das für den Lysin-Export kodierende lysE-Gen (DE-A-195 48 222)
überexprimiert werden.

Weiterhin kann es für die Produktion von Aminosäuren insbesondere L-Lysin, vorteilhaft sein, neben der Überexpression des eno-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren, insbesondere L-Lysin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an Lysin gebildet hat. Dieses Ziel wird normalerweise innerhalb von 10 Stunden bis 160 Stunden erreicht.

Die Analyse von L-Lysin erfolgt kann durch Anionenaustauschchromatographie mit anschließender Ninhydrin Derivatisierung erfolgen so wie bei Spackman et al. (Analytical Chemistry, 30, (1958), 1190) beschrieben.

Das erfindungsgemäße Verfahren dient zur fermentativen Herstellung von Aminosäuren, insbesondere L-Lysin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al., (1995, Plasmid 33:168-179) beschrieben, isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

### Isolierung und Sequenzierung des eno-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID NO 1 dargestellt. Die Analyse der Nukleotidsequenz ergab ein offenes Leseraster von 1275 Basenpaaren, welches als eno-Gen bezeichnet wurde. Das eno-Gen kodiert für ein Protein von 425 Aminosäuren.

### Beispiel 3

### Herstellung eines Shuttlevektors pXT-enoex zur Verstärkung des eno-Gens in C. glutamicum

### 3.1. Klonierung des eno-Gens

Aus dem Stamm ATCC 13032 wurde nach der Methode von Eikmanns et al. (Microbiology 140: 1817-1828 (1994)) chromosomale DNA isoliert. Aufgrund der aus Beispiel 2 für C. glutamicum bekannten Sequenz des eno-Gens wurden die folgenden Oligonukleotide für die Polymerase Kettenreaktion ausgewählt:
eno-ex1:
   5 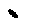 TTG GCA TAG GAG GCC ACA GT 3 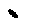
eno-ex2:
   5 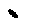 ATT TAG CCC TGA AAG CGT GG 3 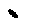

Die dargestellten Primer wurden von der Firma ARK Scientific GmbH Biosystems (Darmstadt, Deutschland) synthetisiert und nach der Standard-PCR-Methode von Innis et al. (PCR protocols. A guide to methods and applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion ermöglichen die Primer die Amplifikation eines ca. 1,3 kb großen DNA-Fragmentes, welches das eno-Gen trägt. Die DNA-Sequenz des amplifizierten DNA Fragmentes wurde durch Sequenzierung überprüft.

### 3.2. Herstellung des E. coli - C. glutamicum Shuttle Vektors pEC-XT99A

Als Ausgangsvektor zur Konstruktion des E. coli-C. glutamicum-Shuttle-Expressionsvektors pEC-XT99A wurde der E.coli-Expressionsvektor pTRC99A (Amann et al. 1988, Gene 69:301-315) verwendet. Nach BspHI-Restriktionsspaltung (Roche Diagnostics GmbH, Mannheim, Deutschland, Produktbeschreibung BspHI, Product No. 1467123) und anschließender Klenow-Behandlung (Amersham Pharmacia Biotech, Freiburg, Deutschland, Produktbeschreibung Klenow Fragment of DNA Polymerase I, Product No. 27-0928-01; Methode nach Sambrook et al., 1989, Molecular Cloning: A laboratory Manual, Cold Spring Harbor) wurde das Ampicillin-Restenzgen (bla) gegen das Tetracyclin-Resistenzgen des C. glutamicum Plasmids pAG1 (GenBank Accession No. AF121000) ausgetauscht. Hierzu wurde der Resistenzgen-tragende Bereich als AluI-Fragment (Amersham Pharmacia Biotech, Freiburg, Deutschland, Produktbeschreibung AluI, Product No. 27-0884-01) in den linearisierten E.coli-Expressionsvektor pTRC99A kloniert. Die Ligation wurde wie von Sambrook et al. (1989, Molecular Cloning: A laboratory Manual, Cold Spring Harbor) beschrieben durchgeführt, wobei das DNA-Gemisch mit T4-Ligase (Amersham Pharmacia Biotech, Freiburg, Deutschland, Produktbeschreibung T4-DNA-Ligase, Product No. 27-0870-04) über Nacht inkubiert wurde. Dieses Ligationsgemisch wurde anschließend in den E. coli-Stamm DH5αmcr (Grant, 1990, Proceedings of the National Academy of Sciences U.S.A., 87:4645-4649) elektroporiert (Tauch et al. 1994, FEMS Microbiology Letters, 123:343-7). Der konstruierte E. coli-Expressionsvektor wurde mit pXT99A bezeichnet.

Als Basis zur Klonierung eines Minimalreplikons aus Corynebacterium glutamicum wurde das Plasmid pGA1 (Sonnen et al. 1991, Gene, 107:69-74) verwandt. Durch BalI/PstI-Restriktionsspaltung (Promega GmbH, Mannheim, Deutschland, Produktbeschreibung BalI, Product No. R6691; Amersham Pharmacia Biotech, Freiburg, Deutschland Produktbeschreibung PstI, Product No. 27-0976-01) des Vektors pGA1 konnte ein 3484 bp großes Fragment in den mit SmaI und PstI (Amersham Pharmacia Biotech, Freiburg, Deutschland, Produktbeschreibung SmaI, Product No. 27-0942-02, Produktbeschreibung PstI, Product No. 27-0976-01) fragmentierten Vektor pK18mob2 (Tauch et al., 1998, Archives of Microbiology 169: 303-312) kloniert werden. Mittels BamHI/XhoI-Restriktionsspaltung (Amersham Pharmacia Biotech, Freiburg, Deutschland, Produktbeschreibung BamHI, Product No. 27-086803, Produktbeschreibung XhoI, Product No. 27-0950-01) und anschließender Klenow-Behandlung (Amersham Pharmacia Biotech, Freiburg, Deutschland, Produktbeschreibung Klenow Fragment of DNA Polymerase I, Product No. 27-0928-01; Methode nach Sambrook et al., 1989, Molecular Cloning: A laboratory Manual, Cold Spring Harbor) wurde ein 839 bp großes Fragment deletiert. Aus dem mit T4-Ligase (Amersham Pharmacia Biotech, Freiburg, Deutschland, Produktbeschreibung T4-DNA-Ligase, Product No. 27-0870-04) religierten Konstrukt konnte das C. glutamicum Minimalreplikon als 2645 bp großes Fragment in den E.coli-Expressionsvektor pXT99A kloniert werden. Hierzu wurde die DNA des Minimalreplikon-tragenden Konstruktes mit den Restriktionsenzymen KpnI (Amersham Pharmacia Biotech, Freiburg, Deutschland, Produktbeschreibung KpnI, Product No. 27-0908-01) und PstI (Amersham Pharmacia Biotech, Freiburg, Deutschland, Produktbeschreibung PstI, Product No. 27-0886-03) gespalten und anschließend mittels Klenow-Polymerase (Amersham Pharmacia Biotech, Freiburg, Deutschland, Produktbeschreibung Klenow Fragment of DNA Polymerase I, Product No. 27-0928-01) eine 3'-5'-Exonukleasebehandlung (Sambrook et al., 1989, Molecular Cloning: A laboratory Manual, Cold Spring Harbor) durchgeführt.

In einem parallelen Ansatz wurde der E.coli-Expressionsvektor pXT99A mit dem Restriktionsenzym RsrII (Roche Diagnostics, Mannheim, Deutschland, Produktbeschreibung RsrII, Product No. 1292587) gespalten und mit Klenow-Polymerase (Amersham Pharmacia Biotech, Freiburg, Deutschland, Klenow Fragment of DNA Polymerase I, Product No. 27-0928-01) zur Ligation vorbereitet. Die Ligation des Minimalreplikons mit dem Vektorkonstrukt pXT99A wurde wie von Sambrook et al. (1989, Molecular Cloning: A laboratory Manual, Cold Spring Harbor) beschrieben durchgeführt, wobei das DNA-Gemisch mit T4-Ligase (Amersham Pharmacia Biotech, Freiburg, Deutschland, Produktbeschreibung T4-DNA-Ligase, Product No. 27-0870-04) über Nacht inkubiert wurde.

Der so konstruierte E. coli-C. glutamicum-Shuttle-Expressionsvektor pEC-XT99A wurde mittels Elektroporation (Liebl et al., 1989, FEMS Microbiology Letters, 53:299-303) in C. glutamicum DSM5715 transferiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 5 mg/l Tetracyclin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit der Restriktionsendonuklease HindIII geschnitten und das Plasmid durch anschließende Agarosegel-Elektrophorese überprüft.

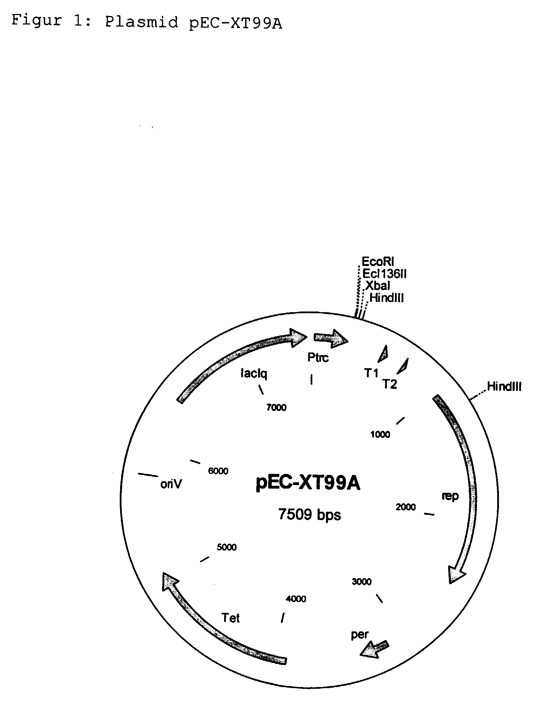

Das so erhaltene Plasmidkonstrukt wurde als pEC-XT99A bezeichnet und ist in Figur 1 dargestellt. Der durch Elektroporation des Plasmides pEC-XT99A in den Corynebacterium glutamicum-Stamm DSM5715 erhaltene Stamm wurde DSM5715/pEC-XT99A genannt und als DSM 12967 bei der Deutschen Sammlung für Mikroorganismen und Zellkulturen (DSMZ, Braunschweig, Deutschland) gemäß Budapester Vertrag hinterlegt.

### 3.3. Klonierung von eno im E. coli-C. glutamicum Shuttle Vektor pEC-XT99A

Als Vektor wurde der in Beispiel 3.2 beschriebene E. coli - C. glutamicum Shuttle-Vektor pEC-XT99A verwendet. DNA dieses Plasmids wurde mit dem Restriktionsenzym Ecl136II vollständig gespalten und anschließend mit shrimp alkalischer Phosphatase (Roche Diagnostics GmbH, Mannheim, Deutschland, Produktbeschreibung SAP, Product No. 1758250) dephosphoryliert.

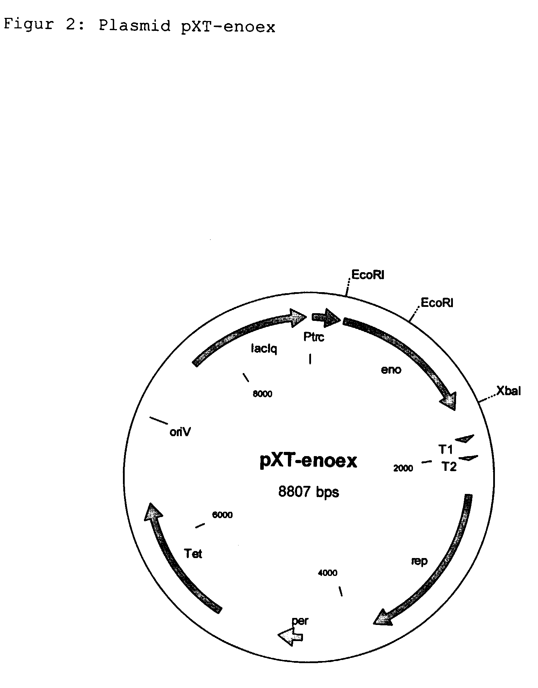

Das wie in Beispiel 3.1 beschrieben gewonnene eno-Fragment wurde mit dem vorbereiteten Vektor pEC-XT99A gemischt und der Ansatz mit T4-DNA-Ligase (Amersham Pharmacia, Freiburg, Deutschland, Produktbeschreibung T4-DNA-Ligase, Code no.27-0870-04) behandelt. Der Ligationsansatz wurde in den E. coli Stamm DH5αmcr (Grant, 1990, Proceedings of the National Academy of Sciences U.S.A., 87:4645-4649) transformiert. Die Selektion von Plasmid-tragenden Zellen erfolgte durch Ausplattieren des Transformationsansatzes auf LB-Agar (Lennox, 1955, Virology, 1:190) mit 5 mg/l Tetracyclin. Nach Inkubation über Nacht bei 37°C wurden rekombinante Einzelklone selektioniert. Plasmid DNA wurde aus einer Transformante mit dem Qiaprep Spin Miniprep Kit (Product No. 27106, Qiagen, Hilden, Germany) nach Herstellerangaben isoliert und mit den Restriktionsenzymen EcoRI und XbaI gespalten, um das Plasmid durch anschließende Agarosegel-Elektrophorese zu überprüfen. Das erhaltene Plasmid wurde pXT-enoex genannt und ist in Figur 2 dargestellt.

### Beispiel 4:

### Transformation des Stammes DSM5715 mit dem Plasmid pXT-enoex

Der Stamm DSM5715 wurde mit dem Plasmid pXT-enoex unter Anwendung der von Liebl et al., (FEMS Microbiology Letters, 53:299-303 (1989)) beschriebenen Elektroporationsmethode transformiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 5 mg/l Tetracyclin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit den Restriktionsendonukleasen EcoRI und XbaI geschnitten und das Plasmid durch anschließende Agarosegel-Elektrophorese überprüft. Der erhaltene Stamm wurde DSM5715/pXT-enoex genannt.

### Beispiel 5:

### Herstellung von Lysin

Der in Beispiel 4 erhaltene C. glutamicum Stamm DSM5715/pXT-enoex wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz-Agar mit Tetracyclin (5 mg/l)) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet.

### Medium Cg III

| | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |
| Der pH-Wert wurde auf pH 7.4 eingestellt | |

Diesem wurde Tetracyclin (5 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,05 betrug. Für die Hauptkultur wurde das Medium MM verwendet.

### Medium MM

| | |
|---|---|
| CSL (Corn Steep Liquor) | 5 g/l |
| MOPS (Morpholinopropansulfonsäure) | 20 g/l |
| Glucose (getrennt autoklaviert) | 100 g/l |
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| L-Leucin (sterilfiltriert) | 0,1 g/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Tetracyclin (5 mg/l) zugesetzt. Die Kultivierung erfolgte bei 33°C und 80% Luftfeuchte.

Nach 72 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| DSM5715/pEC-XT99A | 7,4 | 15,5 |
| DSM5715/pXT-enoex | 7,5 | 16,5 |

Folgende Figuren sind beigefügt:
Figur 1: Karte des Plasmids pEC-XT99A
Figur 2: Karte des Plasmids pXT-enoex

Die verwendeten Abkürzungen und Bezeichnungen haben folgende Bedeutung.
- per:: Gen zur Kontrolle der Kopienzahl aus pGA1
- oriV:: ColE1-ähnlicher Origin aus pMB1
- rep:: Plasmidkodierter Replikationsursprung aus C. glutamicum Plasmid pGA1
- Ptrc:: trc-Promotor aus pTRC99A
- T1, T2:: Terminatorregionen 1 und 2 aus pTRC99A
- lacIq:: Repressor-Gen des Lac-Operon
- Tet:: Resistenzgen für Tetracyclin
- eno:: Enolase-Gen eno von C.glutamicum
- EcoRI:: Schnittstelle des Restriktionsenzyms EcoRI
- Ecl136II:: Schnittstelle des Restriktionsenzyms Ecl136II
- HindIII:: Schnittstelle des Restriktionsenzyms HindIII
- XbaI:: Schnittstelle des Restriktionsenzyms XbaI

## Patentansprüche

1. Isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
e) Polynukleotid, enthaltend mindestens 15 aufeinanderfolgende Basen der Polynukleotidsequenz von a), b) oder c).

2. Polynukleotid gemäß Anspruch 1,
wobei das Polynukleotid eine in coryneformen Bakterien replizierbare, bevorzugt rekombinante DNA ist.

3. Polynukleotid gemäß Anspruch 1,
wobei das Polynukleotid eine RNA ist.

4. Polynukleotid gemäß Anspruch 2,
enthaltend die Nukleinsäuresequenz wie in SEQ ID No. 1 dargestellt.

5. Replizierbare DNA gemäß Anspruch 2,
enthaltend
(i) die Nukleotidsequenz, gezeigt in SEQ-ID-No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

6. Polynukleotidsequenz gemäß Anspruch 2, das für ein Polypeptid codiert, das die Aminosäuresequenz in SEQ ID No. 2 darstellt, enthält.

7. Verfahren zur fermentativen Herstellung von L-Aminosäuren, insbesondere L-Lysin,
**dadurch gekennzeichnet**,
daß man folgende Schritte durchführt:
a) Fermentation der die L-Lysin produzierenden coryneformen Bakterien, in denen man zumindest das eno-Gen oder dafür codierende Nukleotidsequenzen verstärkt, insbesondere überexprimiert.
b) Anreicherung von der L-Aminosäure im Medium oder in den Zellen der Bakterien und
c) Isolieren von der L-Aminosäure.

8. Verfahren gemäß Anspruch 7,
**dadurch gekennzeichnet**,
daß man Bakterien einsetzt, in denen man zusätzlich weitere Gene des Biosyntheseweges der gewünschten L-Aminosäure verstärkt.

9. Verfahren gemäß Anspruch 7,
**dadurch gekennzeichnet**,
daß man Bakterien einsetzt, in denen die Stoffwechselwege zumindest teilweise ausgeschaltet sind, die die Bildung des L-Lysins verringern.

10. Verfahren gemäß Anspruch 7,
**dadurch gekennzeichnet**,
daß man einen mit einem Plasmidvektor transformierten Stamm einsetzt, und der Plasmidvektor die für das eno-Gen codierende Nukleotidsequenz trägt.

11. Verfahren gemäß einem oder mehreren der Ansprüche 7 bis 10,
**dadurch gekennzeichnet**,
daß man coryneforme Bakterien verwendet, die L-Lysin herstellen.

12. Verfahren gemäß Anspruch 8,
**dadurch gekennzeichnet**,
daß gleichzeitig das für die Dihydrodipicolinat-Synthase kodierende dapA-Gen überexprimiert wird.

13. Verfahren gemäß Anspruch 8,
**dadurch gekennzeichnet**,
daß gleichzeitig ein S-(2-Aminoethyl)-Cystein-Resistenz vermittelndes DNA-Fragment amplifiziert wird.

14. Verfahren gemäß Anspruch 8,
**dadurch gekennzeichnet**,
daß gleichzeitig das für die Gyceraldehyd-3-Phosphat Dehydrogenase kodierende gap-Gen überexprimiert wird.

15. Verfahren gemäß Anspruch 8,
**dadurch gekennzeichnet**,
daß gleichzeitig das für die Triosephosphat Isomerase codierende tpi-Gen überexprimiert wird.

16. Verfahren gemäß Anspruch 8,
**dadurch gekennzeichnet**,
daß gleichzeitig das für die 3-Phosphatglycerat Kinase codierende pgk-Gen überexprimiert wird.

17. Verfahren gemäß Anspruch 8,
**dadurch gekennzeichnet**,
daß gleichzeitig das für die Pyruvat Carboxylase codierende pyc-Gen überexprimiert wird.


SEQUENZPROTOKOLL

<110> Degussa-Hüls AG

<120> Neue für das eno-Gen codierende Nukleotidsequenzen

<130> 990152 BT

<140>
<141>

<160> 2

<170> PatentIn Ver. 2.1

<210> 1
<211> 1578
<212> DNA
<213> Corynebacterium glutamicum

<220>
<221> CDS
<222> (151)..(1425)

<400> 1
ggctggggat atgggtagtt ttcgccacta atttcaactg attgcctcat cgaaacaaga 60

ttcgtgcaac aattgggtgt agacgtgatt gaagacattt gatcacgtga ataattctag 120

ttagctccca agttggcata ggaggccaca gtg gct gaa atc atg cac gta ttc 174
Val Ala Glu Ile Met His Val Phe
1               5

gct cgc gaa att ctc gac tcc cgc ggt aac cca acc gtc gag gca gag 222
Ala Arg Glu Ile Leu Asp Ser Arg Gly Asn Pro Thr Val Glu Ala Glu
    10                  15                  20

gtt ttc ctg gat gac ggt tcc cac ggt gtc gca ggt gtt cca tcc ggc 270
Val Phe Leu Asp Asp Gly Ser His Gly Val Ala Gly Val Pro Ser Gly
25                  30                  35                  40

gca tcc acc ggc gtc cac gag gct cat gag ctg cgt gac ggt ggc gat 318
Ala Ser Thr Gly Val His Glu Ala His Glu Leu Arg Asp Gly Gly Asp
                45                  50                  55

cgc tac ctg ggc aag ggc gtt ttg aag gca gtt gaa aac gtc aac gaa 366
Arg Tyr Leu Gly Lys Gly Val Leu Lys Ala Val Glu Asn Val Asn Glu
            60                  65                  70

gaa atc ggc gac gag ctc gct ggc cta gag gct gac gat cag cgc ctc 414
Glu Ile Gly Asp Glu Leu Ala Gly Leu Glu Ala Asp Asp Gln Arg Leu
        75                  80                  85

atc gac gaa gca atg atc aag ctt gat ggc acc gcc aac aag tcc cgc 462
Ile Asp Glu Ala Met Ile Lys Leu Asp Gly Thr Ala Asn Lys Ser Arg
    90                  95                  100

ctg ggt gca aac gca atc ctt ggt gtt tcc atg gct gtt gca aag gct     510
Leu Gly Ala Asn Ala Ile Leu Gly Val Ser Met Ala Val Ala Lys Ala
105                 110                 115                 120

gct gct gat tcc gca ggc ctc cca ctg ttc cgc tac atc ggt gga cca     558
Ala Ala Asp Ser Ala Gly Leu Pro Leu Phe Arg Tyr Ile Gly Gly Pro
                125                 130                 135

aac gca cac gtt ctt cca gtt cca atg atg aac atc atc aac ggt ggc     606
Asn Ala His Val Leu Pro Val Pro Met Met Asn Ile Ile Asn Gly Gly
            140                 145                 150

gct cac gct gac tcc ggt gtt gac gtt cag gaa ttc atg atc gct cca     654
Ala His Ala Asp Ser Gly Val Asp Val Gln Glu Phe Met Ile Ala Pro
        155                 160                 165

atc ggt gca gag acc ttc tct gag gct ctc cgc aac ggc gcg gag gtc     702
Ile Gly Ala Glu Thr Phe Ser Glu Ala Leu Arg Asn Gly Ala Glu Val
    170                 175                 180

tac cac gca ctg aag tcc gtc atc aag gaa aag ggc ctg tcc acc gga     750
Tyr His Ala Leu Lys Ser Val Ile Lys Glu Lys Gly Leu Ser Thr Gly
185                 190                 195                 200

ctt ggc gat gag ggc ggc ttc gct cct tcc gtc ggc tcc acc cgt gag     798
Leu Gly Asp Glu Gly Gly Phe Ala Pro Ser Val Gly Ser Thr Arg Glu
                205                 210                 215

gct ctt gac ctt atc gtt gag gca atc gag aag gct ggc ttc acc cca     846
Ala Leu Asp Leu Ile Val Glu Ala Ile Glu Lys Ala Gly Phe Thr Pro
            220                 225                 230

ggc aag gac atc gct ctt gct ctg gac gtt gct tcc tct gag ttc ttc     894
Gly Lys Asp Ile Ala Leu Ala Leu Asp Val Ala Ser Ser Glu Phe Phe
        235                 240                 245

aag gac ggc acc tac cac ttc gaa ggt ggc cag cac tcc gca gct gag     942
Lys Asp Gly Thr Tyr His Phe Glu Gly Gly Gln His Ser Ala Ala Glu
    250                 255                 260

atg gca aac gtt tac gct gag ctc gtt gac gcg tac cca atc gtc tcc     990
Met Ala Asn Val Tyr Ala Glu Leu Val Asp Ala Tyr Pro Ile Val Ser
265                 270                 275                 280

atc gag gac cca ctg cag gaa gat gac tgg gag ggt tac acc aac ctc     1038
Ile Glu Asp Pro Leu Gln Glu Asp Asp Trp Glu Gly Tyr Thr Asn Leu
                285                 290                 295

acc gca acc atc ggc gac aag gtt cag atc gtt ggc gac gac ttc ttc     1086
Thr Ala Thr Ile Gly Asp Lys Val Gln Ile Val Gly Asp Asp Phe Phe
            300                 305                 310

gtc acc aac cct gag cgc ctg aag gag ggc atc gct aag aag gct gcc     1134
Val Thr Asn Pro Glu Arg Leu Lys Glu Gly Ile Ala Lys Lys Ala Ala
        315                 320                 325

aac tcc atc ctg gtt aag gtg aac cag atc ggt acc ctc acc gag acc     1182
Asn Ser Ile Leu Val Lys Val Asn Gln Ile Gly Thr Leu Thr Glu Thr
    330                 335                 340

ttc gac gct gtc gac atg gct cac cgc gca ggc tac acc tcc atg atg     1230
Phe Asp Ala Val Asp Met Ala His Arg Ala Gly Tyr Thr Ser Met Met
345                 350                 355                 360

tcc cac cgt tcc ggt gag acc gag gac acc acc att gct gac ctc gca     1278
Ser His Arg Ser Gly Glu Thr Glu Asp Thr Thr Ile Ala Asp Leu Ala
                365                 370                 375

gtt gca ctc aac tgt ggc cag atc aag act ggt gct cca gca cgt tcc     1326
Val Ala Leu Asn Cys Gly Gln Ile Lys Thr Gly Ala Pro Ala Arg Ser
            380                 385                 390

gac cgt gtc gca aag tac aac cag ctt ctc cgc atc gag cag ctg ctt     1374
Asp Arg Val Ala Lys Tyr Asn Gln Leu Leu Arg Ile Glu Gln Leu Leu
        395                 400                 405

ggc gac gcc ggc gtc tac gca ggt cgc agc gca ttc cca cgc ttt cag     1422
Gly Asp Ala Gly Val Tyr Ala Gly Arg Ser Ala Phe Pro Arg Phe Gln
    410                 415                 420

ggc taaataaaag cgcttttcga cgcccggtaa cctcaaggtt gccgggcgtc          1475
Gly
425

gttgccttac tactgttact ggtgtgacta tgatcgagga ttatggcaaa gcagaagaaa 1535

actcataaag gccttgttcc tgtctcaagc agggaacgtg ctt                     1578


<210> 2
<211> 425
<212> PRT
<213> Corynebacterium glutamicum

<400> 2
Val Ala Glu Ile Met His Val Phe Ala Arg Glu Ile Leu Asp Ser Arg
  1               5                  10                  15

Gly Asn Pro Thr Val Glu Ala Glu Val Phe Leu Asp Asp Gly Ser His
             20                  25                  30

Gly Val Ala Gly Val Pro Ser Gly Ala Ser Thr Gly Val His Glu Ala
         35                  40                  45

His Glu Leu Arg Asp Gly Gly Asp Arg Tyr Leu Gly Lys Gly Val Leu
     50                  55                  60

Lys Ala Val Glu Asn Val Asn Glu Glu Ile Gly Asp Glu Leu Ala Gly
 65                  70                  75                  80

Leu Glu Ala Asp Asp Gln Arg Leu Ile Asp Glu Ala Met Ile Lys Leu
                 85                  90                  95

Asp Gly Thr Ala Asn Lys Ser Arg Leu Gly Ala Asn Ala Ile Leu Gly
            100                 105                 110

Val Ser Met Ala Val Ala Lys Ala Ala Ala Asp Ser Ala Gly Leu Pro
        115                 120                 125

Leu Phe Arg Tyr Ile Gly Gly Pro Asn Ala His Val Leu Pro Val Pro
    130                 135                 140

Met Met Asn Ile Ile Asn Gly Gly Ala His Ala Asp Ser Gly Val Asp
145                 150                 155                 160

Val Gln Glu Phe Met Ile Ala Pro Ile Gly Ala Glu Thr Phe Ser Glu
                165                 170                 175

Ala Leu Arg Asn Gly Ala Glu Val Tyr His Ala Leu Lys Ser Val Ile
            180                 185                 190

Lys Glu Lys Gly Leu Ser Thr Gly Leu Gly Asp Glu Gly Gly Phe Ala
        195                 200                 205

Pro Ser Val Gly Ser Thr Arg Glu Ala Leu Asp Leu Ile Val Glu Ala
    210                 215                 220

Ile Glu Lys Ala Gly Phe Thr Pro Gly Lys Asp Ile Ala Leu Ala Leu
225                 230                 235                 240

Asp Val Ala Ser Ser Glu Phe Phe Lys Asp Gly Thr Tyr His Phe Glu
                245                 250                 255

Gly Gly Gln His Ser Ala Ala Glu Met Ala Asn Val Tyr Ala Glu Leu
            260                 265                 270

Val Asp Ala Tyr Pro Ile Val Ser Ile Glu Asp Pro Leu Gln Glu Asp
        275                 280                 285

Asp Trp Glu Gly Tyr Thr Asn Leu Thr Ala Thr Ile Gly Asp Lys Val
    290                 295                 300

Gln Ile Val Gly Asp Asp Phe Phe Val Thr Asn Pro Glu Arg Leu Lys
305                 310                 315                 320

Glu Gly Ile Ala Lys Lys Ala Ala Asn Ser Ile Leu Val Lys Val Asn
                325                 330                 335

Gln Ile Gly Thr Leu Thr Glu Thr Phe Asp Ala Val Asp Met Ala His
            340                 345                 350

Arg Ala Gly Tyr Thr Ser Met Met Ser His Arg Ser Gly Glu Thr Glu
        355                 360                 365

Asp Thr Thr Ile Ala Asp Leu Ala Val Ala Leu Asn Cys Gly Gln Ile
    370                 375                 380

Lys Thr Gly Ala Pro Ala Arg Ser Asp Arg Val Ala Lys Tyr Asn Gln
385                 390                 395                 400

Leu Leu Arg Ile Glu Gln Leu Leu Gly Asp Ala Gly Val Tyr Ala Gly
                405                 410                 415

Arg Ser Ala Phe Pro Arg Phe Gln Gly
            420                 425